# EUROPÄISCHE PATENTSCHRIFT

(11) **EP 0 868 884 B1**
(45) Veröffentlichungstag und Bekanntmachung des Hinweises auf die Patenterteilung: **15.06.2005**
(21) Anmeldenummer: 98104882.0
(22) Anmeldetag: 18.03.1998
(51) Int. Cl.: A61B 18/12

(54) **Vorrichtung und Verfahren zur Katheterablation**
Apparatus and method for catheter ablation
Méthode et dispositif pour ablation avec un cathéter

(30) Priorität: 01.04.1997 DE 19713527; 22.05.1997 DE 19721362
(43) Veröffentlichungstag der Anmeldung: 07.10.1998
(73) Patentinhaber: Muntermann, Axel, 35578 Wetzlar (DE)
(72) Erfinder: Muntermann, Axel, 35578 Wetzlar (DE)
(74) Vertreter: Zwirner, Gottfried, Dipl.-Ing. Dipl.-W.-Ing.

(56) Entgegenhaltungen:
- WO-A-94/10922
- WO-A-96/34569
- DE-U- 29 519 651

## Beschreibung

Die Erfindung betrifft eine Vorrichtung zur Katheterablation im allgemeinen und zur Radiofrequenz-Katheterablation von vorzugsweise lebendem endomyokardialem Gewebe im speziellen, sowie ein entsprechendes Ablationskatheter.

Bei der Behandlung von Herzrhythmusstörungen, welche durch elektrisch autonomes Gewebe und insbesondere durch Gewebe, welches der zentralen Erregungssteuerung des Herzens nicht unterworfen ist, erzeugt werden, hat sich das thermische Koagulieren des betreffenden Gewebes als erfolgreich herausgestellt und ist auf dem Gebiet gut eingeführt. Dabei wird der Ablationskatheter endocardial in das Herz geschoben und von einer Elektrode an der Spitze des Katheters oder von entlang der Katheterlängsachse angeordneten Elektroden ausgehende Energie an das betreffende Gewebe abgegeben, sodaß eine lokale Koagulation auftritt und der die Störung verursachende Gewebebereich elektrisch isoliert wird. Üblicherweise wird hierbei die Energie kontinuierlich mittels eines Hochfrequenzgenerators mit Frequenzen von 300 KHz bis 700 KHz an die Elektrode abgegeben und so eine Koagulationsnarbe bis zu einer Tiefe von etwa 3 bis 5 mm erzeugt.

Nachteilig ist diesem Verfahren jedoch, daß die Temperatur des Katheters teilweise derart hoch ist, daß sich koagulierendes Blut bildet und am Katheter haftet. Hierdurch wird das weitere Abladieren verhindert und ein Säubern der Elektrode notwendig. Das nachfolgende Herausziehen und wiederholte Einschieben des Katheters bedeutet sowohl für den Patienten als auch für den Operateur eine hohe zusätzliche Belastung. Weiterhin ergibt sich durch das Koagulierte Blut eine hohe Thrombosegefahr. Andererseits besteht ein Bedarf, die an das Gewebe abgegebene Energie weiter zu erhöhen, um mit größeren Läsionstiefen auch tief im Myocard liegende arythmogene Gewebebereiche zu erreichen.

Das Dokument WO-A-96/34569 offenbart Vorrichtungen gemäß Oberbegriff der Ansprüche 1 und 10.

Der Erfindung liegt die Aufgabe zugrunde, derartige Nachteile bei der Katheterablation von Gewebe zumindest zu mildern und die Energieabgabe an das zu abladierende Gewebe zu steuern, so daß eine vorgegebene Koagulationstemperatur innerhalb des betreffenden Gewebebereichs erreicht wird.

Die Erfindung wird in den Ansprüchen 1 und 10 definiert, jedes Ausführungsbeispiel das im Widerspruch zu mindestens einem der Ansprüche 1 und 10 steht, ist nicht Bestandteil der Erfindung.

Es wurde festgestellt, daß bei gepulsten Ablationsverfahren bei höheren Leistungen, d.h. bei Leistungen von oberhalb von etwa 70 Watt und Kathetertemperaturen von über 60 °C die Gewebeinnentemperatur deutlich höher als die Kathetertemperatur sein kann und es sogar zur Dampfblasenbildung bzw. Vaporisation innerhalb des Gewebes kommen kann, welches unter Umständen letale Folgen nach sich ziehen kann.

Weiterhin kann die Elektrodentemperatur selbst bei hohen Gewebinnentemperaturen in einem Bereich gehalten werden, in welchem Blut nicht an der Elektrode koaguliert und anhaftet. Dabei wird von der Erkenntnis Gebrauch gemacht, daß bei der Verwendung von gepulst betriebenen Ablationskatheter bei einer vorgegebenen maximalen Elektrodentemperatur eine viel größere Energieabgabe an das betreffende Gewebe im Vergleich zu einem kontinuierlich betriebenen Ablationskatheter erfolgt.

Beispielsweise kann bis zum Erreichen der vorgegebenen Elektrodentemperatur von 60°C bei einem gepulst betriebenen Ablationskatheter mit einem Tastverhältnis von eins zu eins in etwa die doppelte Energie an einen Gewebeabschnitt abgegeben werden gegenüber einem kontinuierlich betriebenen Ablationskatheter. Durch die erhöhte Energieabgabe sind weiterhin größere Läsionstiefen erreichbar womit auch im Myocard tiefer gelegene arhythmogene Gewebebereiche erfaßt werden können. Durch die erhöhte Energieabgabe wird schneller die notwendige Läsionstiefe erreicht, hierbei werden sogar Tiefen erreicht, die mit kontinuierlichen Verfahren nicht zu erreichen sind, und es wird dabei der Zeitbedarf herabgesetzt.

Der Katheter kann vorteilhafterweise mehrere selektiv ansteuerbare Elektroden mit zugeordneten thermischen Sensoren umfassen womit eine linienförmige Ablation durchgeführbar ist. Dies hat eine kürzere Behandlungszeit und damit eine verminderte Belastung des Patienten zur Folge und es kann mit einer derartigen Unterbrechung der Reizleitung unter Umständen das Trennen des betreffenden Gewebeabschnittes von der übergeordneten Reizleitung wirkungsvoller als mit herkömmlichen Verfahren sichergestellt werden.

Dem Umstand, daß beim Pulsbetrieb die Gewebeinnentemperatur häufig unkontrolliert hohe Werte erreicht, wird dadurch Rechnung getragen, daß für jeden Ablationskatheter in einem Testaufbau ein Parametersatz mit gleichzeitiger direkter Messung der Gewebeinnentemperatur am Probekörper aufgenommen wird und das Ergebnis als Grundlage für die Berechung der Gewebeinnentemperatur für die nachfolgende Ablation im menschlichen Herz dient. Damit kann im wesentlichen verhindert werden, daß sich unkontrollierte und das Leben des Patienten gefährdende Gewebeinnentemperaturen ausbilden können.

Die Erfindung wird nachfolgend unter Bezugnahme auf bevorzugte Ausführugsformen und anhand der beigefügten Zeichnungen detaillierter beschrieben.
Es zeigen:
- Fig. 1: den Testaufbau zur Ermittlung des Parametersatzes für ein individuelles Ablationskatheter oder einen speziellen Kathetertyp;
- Fig. 2: den Verlauf der Gewebeinnentemperatur für verschiedene maximale Elektrodentemperaturen in Abhängigkeit von der Zeit bei einer kontinuierlichen Leistungs- oder Energieabgabe der Hochfrequenz an die Elektrode bei einer Maximalleistung von 90 Watt;
- Fig. 3: den Verlauf der Gewebeinnentemperatur für verschiedene maximale Elektrodentemperaturen in Abhängigkeit von der Zeit bei einer gepulsten Energieabgabe mit pulsbreitemodulierten Pulsen mit einer maximalen momentanten Leistung von 70 und 110 Watt;
- Fig. 4: den Verlauf der Gewebeinnentemperatur in Abhängigkeit von der Zeit bei einer maximalen Elektrodentemperatur von 60°C für unterschiedliche Fließgeschwindigkeiten einer die Elektrode umgebenden Natriumchloridlösung bei mit kontinuierlicher Hochfrequenzleistung versorgter Katheterelektrode;
- Fig. 5: den Verlauf der Gewebeinnentemperatur in Abhängigkeit von der Zeit bei einer maximalen Elektrodentemperatur von 60°C für unterschiedliche Fließgeschwindigkeiten einer die Elektrode umgebenden Natriumchloridlösung bei mit gepulster Hochfrequenzleistung versorgter Katheterelektrode;
- Fig. 6: die Abhängigkeit der Läsionstiefe von der maximalen Elektrodentemperatur bei verschiedenen Puls-Stromleistungen; und
- Fig. 7: eine tabellarische Zusammenstellung der erfindungsgemäß erreichbaren Läsionswerte in Abhängigkeit von der Kathetertemperatur und der abgegebenen Ablationsleistung im Vergleich zwischen einer kontinuierlich und einer gepulst betriebenen Vorrichtung,
- Fig. 8: einen Abschnitt eines zur Durchführung der Erfindung geeigneten Ablationskatheters mit mehreren Elektroden.

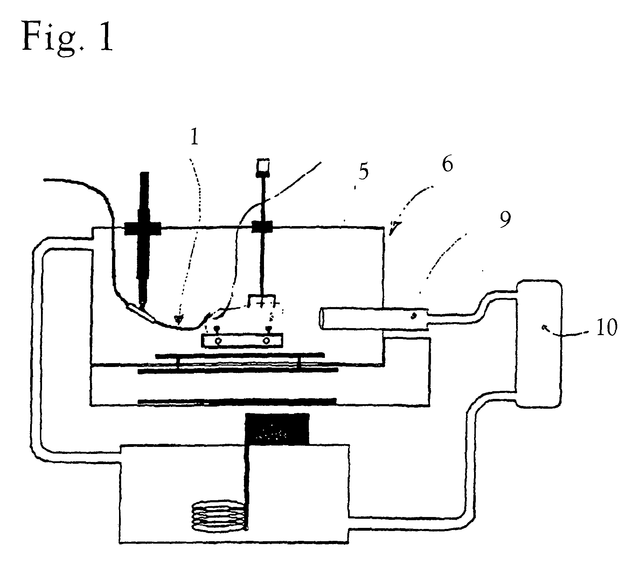

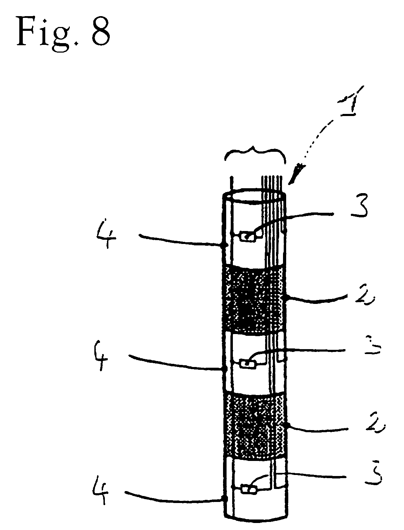

Nachfolgend wird die Erfindung anhand bevorzugter Ausführungsformen und zunächst unter Bezugnahme auf die Fig. 1 und 8 detaillierter beschrieben.

Die erfindungsgemäße Vorrichtung zur Radiofrequenz-Katheterablation umfaßt in an sich bekannter Weise einen Ablationskatheter 1 und einen diesem zugeordneten Anschluß an einen Hochfrequenzgenerator oder ein gesteuertes oder geregeltes Hochfrequenzablationsgerät. Eine derartige Vorrichtung ist beispielsweise in der PCT/DE/00638 beschrieben.

Der Katheter 1 umfaßt zumindest eine Elektrode 2 zum Abladieren von arhythmogenen Gewebe und einen dieser Elektrode zugeordneten thermischen Sensor 3 zur Erfassung der Temperatur der jeweiligen Elektrode 2. Weiterhin umfaßt die Vorrichtung eine in den Figuren nicht dargestellte, der Elektrode zugeordneten Einrichtung zum Ermitteln der Energie, welche an die jeweilige Elektrode 2 abgegeben wird und zumindest einen Sensor 4 sowie eine Einrichtung zur Erfassung der mittleren Fließgeschwindigkeit des Blutes in der Umgebung des Katheters 1 oder alternativ der jeweiligen Elektrode 2.

Der Sensor 4 kann aus einer Lichtleitfaser bestehen, welche in die Flüssigkeit Laserlicht einstrahlt und dopplerverschobene rückgestreute Lichtsignale zurückführt, wobei die Dopplerverschiebung interferometrisch erfassbar ist und je nach Ausbildung und Anordnung der Austrittsöffnungen in der Lichtleitfaser Aufschluß über die lokale oder örtlich gemittelte Strömungsgeschwindigkeit gibt.

Ferner kann der Sensor 4 ein Drucksensor, vorzugsweise ein piezzoelektrischer Staudrucksensor oder ein Ultraschallsensor sein, wobei mit letzerem ebenfalls eine Dopplerverschiebung erfassbar ist.

Vor der Verwendung der erfindungsgemäßen Vorrichtung und des Ablaltionskatheters wird ein Parametersatz für den Ablationskatheter ermittelt, wobei der Katheter 1 an einem Probekörper 5 angewendet wird und wobei in Abhängigkeit von verschiedenen Parameter die Temperatur des zu abladierenden Gewebes innerhalb des Gewebes in der jeweils erwünschten Tiefe, d.h. im jeweils erwünschten Abstand zum Katheter direkt gemessen wird.

Die Gewebeinnentemperatur wird dabei in Abhängigkeit von der Elektrodentemperatur, der an die jeweilige Elektrode abgegebenen Pulsleistung und der mittleren Strömungsgeschwindigkeit einer auf etwa 37°C temperierten Lösung, welche die Elektrode umgibt, ermittelt und als Wertesatz für den jeweiligen individuellen Katheter oder den Kathetertyp abgespeichert. Ferner kann der Wertesatz in erfindungsgemäßer Weise um die Werte der erzielbaren Läsionstiefe erweitert werden.

Fig. 1 stellt den entsprechenden Meßaufbau dar. In der Ablationskammer 6 kann auf dem Präparatschlitten 7 ein Probekörper 5 angebracht werden. Der Ablationskatheter 1 wird an den Probekörper 5 herangebracht und durch Speisen der jeweiligen Elektrode 2 mit einer vorgewählten Impulsleistung eine Ablation bzw. Koagulation von Gewebe erzeugt.

Ein direkt in das Gewebe des Probekörpers 5 bis zur erwünschten Entfernung zum Katheter eingeführter, vorzugsweise punktförmiger Temperatursensor mißt die Gewebeinnentemperatur in Abhängigkeit von der abgegebenen Katheterelektrodenleistung, d.h. der momentante Katheterelektrodenleistung oder der integrierten Katheterelektrodenleistung, in Abhängigkeit von der jeweiligen Katheterelektrodentemperatur und in Abhängikeit von der Fließgeschwindikeit des Blutes bzw. der Lösung innerhalb der Ablationskammer 6. Zur Simulation des fließenden Blutes im Herzen ist eine Düse 9 derart angeordnet, daß der Probekörper 5 und der Katheter 1 von einer aus der Düse 9 ausströmenden Natriumchloridlösung vollständig umflossen wird.

Die Düse 9 wird von einer regelbaren Pumpe 4 gespeist, welche die Lösung auch aus der Kammer 6 transportiert. Die Kammer 6 umfaßt einen geregelten Tauchsieder, der die Temperatur der Lösung vorzugsweise auf eine Körpertemperatur von 37°C hält. Der Kreislauf schließt sich durch den Rückfluß der Lösung aus der Ablationskammer 6 durch die untere geheizte Kammer 11.

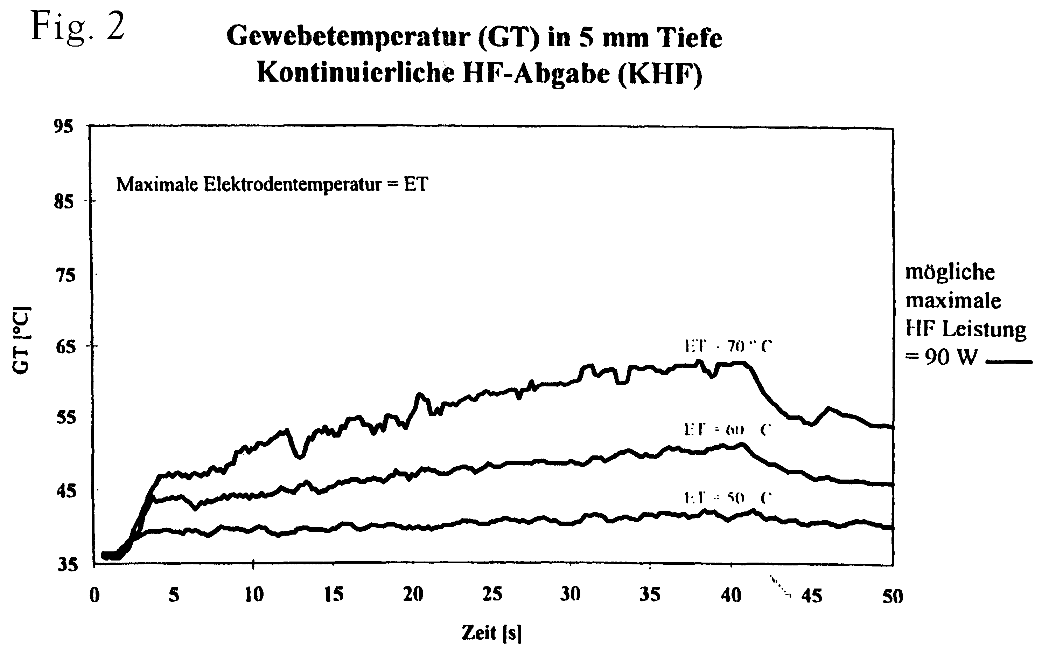

Ein mit dieser Aparatur aufgenommener Verlauf der Gewebeinnentemperatur GT in Abhängigkeit von der Zeit für den Fall einer kontinuierlichen Abgabe der Hochfrequenzenergie für verschiedene maximale Elektrodentemperaturen ET zeigt Fig. 2. Die maximal mögliche HF-Leistung betrug 90 Watt. Wie zu erkennen ist, steigt die Gewebeinnentemperatur bei einer maximal möglichen Elektrodentemperatur von 70°C innerhalb einer Zeit von 40 Sekunden auf etwa 61°C an.

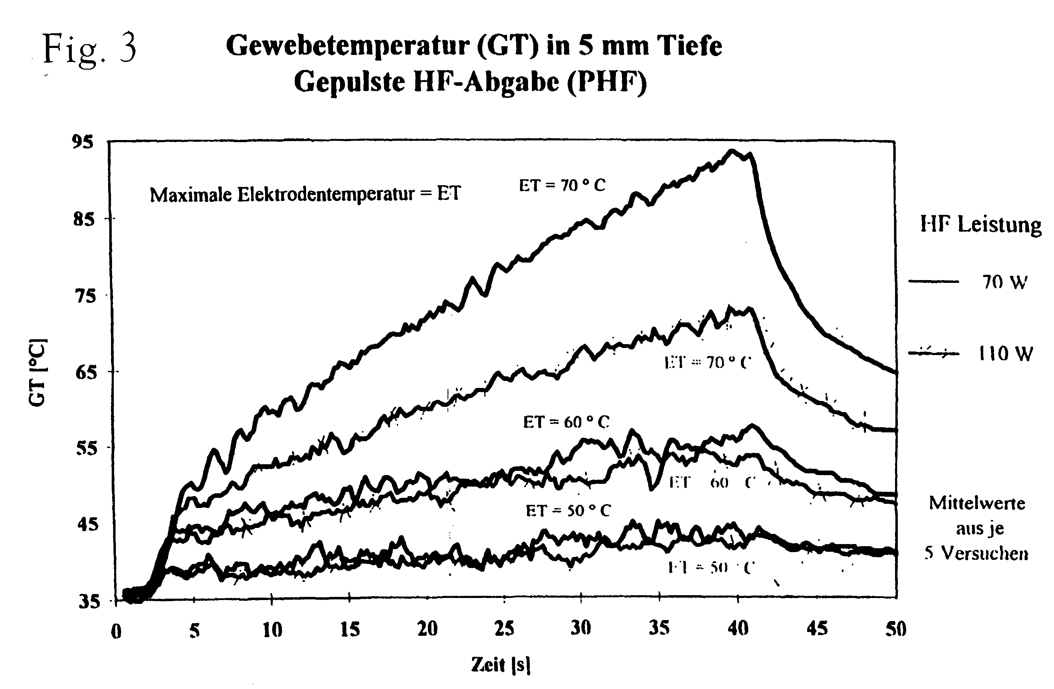

Im Vergleich dazu zeigt die Fig. 3 die gemessene Gewebeinnentemperatur GT in Abhängigkeit von der Zeit bei einer gepulsten HF-Energieabgabe an die Elektrode 2, wiederum wurde die Messung für verschiedene maximale Elektrodentemperaturen ET durchgeführt und aufgezeichnet. Darüberhinaus ist der Verlauf der Gewebeinnentemperatur GT für verschiedene HF-Leistungen aufgetragen.

Es ist deutlich ersichtlich, daß bei einer gepulsten Abgabe der Energie bei einer maximal möglichen Elektrodentemperatur von 70°C ein Ansteigen der Gewebeinnentemperatur bis auf etwa 72°C erreicht wird, wenn die HF-Leistung 70 Watt beträgt.

Bei einer Erhöhung der HF-Leistung auf 110 Watt wird bei gleicher maximaler Elektrodentemperatur von 70°C nach 40 Sekunden in höchst überraschender Weise eine Gewebeinnentemperatur von etwa 93°C, d.h. nahe dem Siedepunkt erreicht. Hier wäre unter Umständen bereits mit Dampfblasenbildung innerhalb des Gewebes zu rechnen. Erstmals durch die vorliegende Erfindung können diese gefährlichen Bereiche erfasst und durch Berücksichtigung der gemessen Werte ausgeschlossen werden.

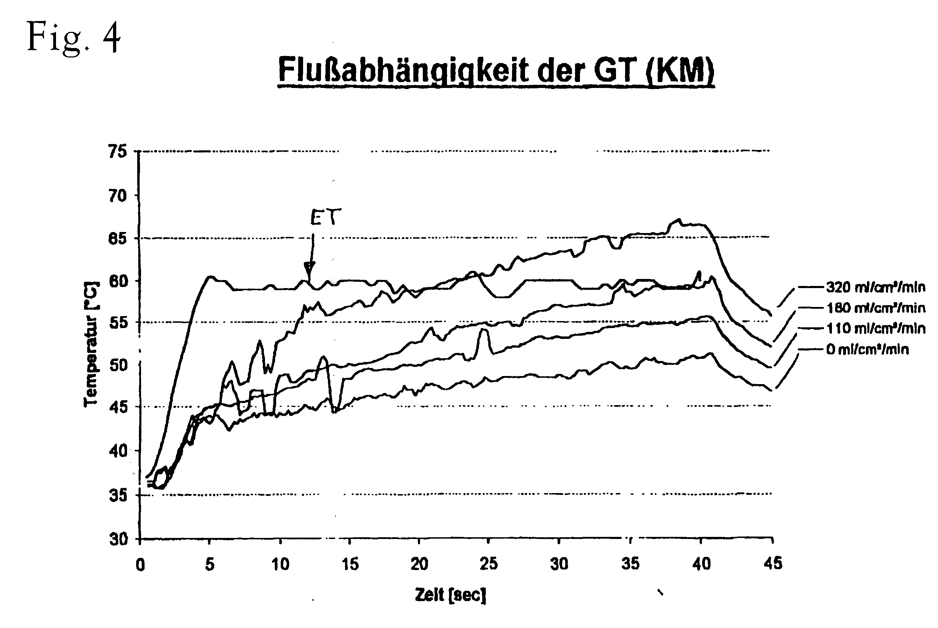

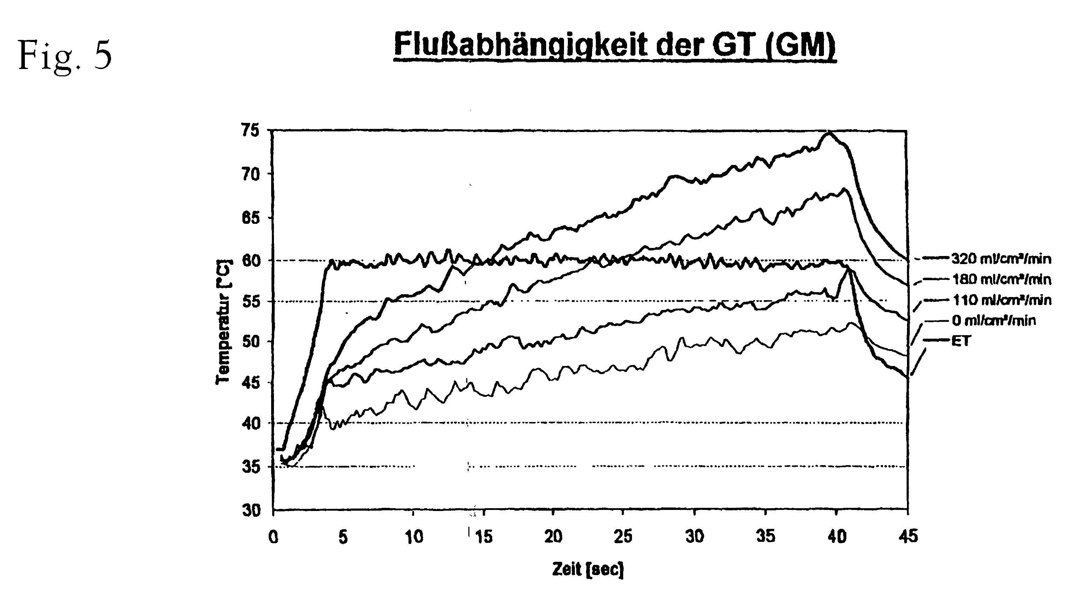

Den Einfluß der Fließgeschwindigkeit der Lösung auf die an das Gewebe des Probekörpers 5 abgegebene Energie zeigen die Fig. 4 und 5. Dort ist die Gewebeinnentemperatur in Abhängigkeit von der Zeit bei verschiedenen Flußgeschwindigkeiten der Lösung dargestellt. Auch hier ist zu erkennen, daß bei gepulster Energieabgabe (Fig. 5) gegenüber der kontinuierlichen Energieabgabe (4) bei jeweils gleicher Katehterelektodentemperatur von etwa 60 °C deutlich höhere Gewebeinnentemperaturen erreicht werden.

Je höher die Geschwindigkeit und damit die Kühlung der Elektrode ist, um so höher ist die abgegebene Leistung und damit die maximal erreichbare Gewebeinnentemperatur, die beispielsweise bei einer Fließgeschwindigkeit, bzw. einem Fluß von 320 ml pro cm² pro Minute nach 40 Sekunden gemäß Fig. 5 etwa 75°C beträgt.

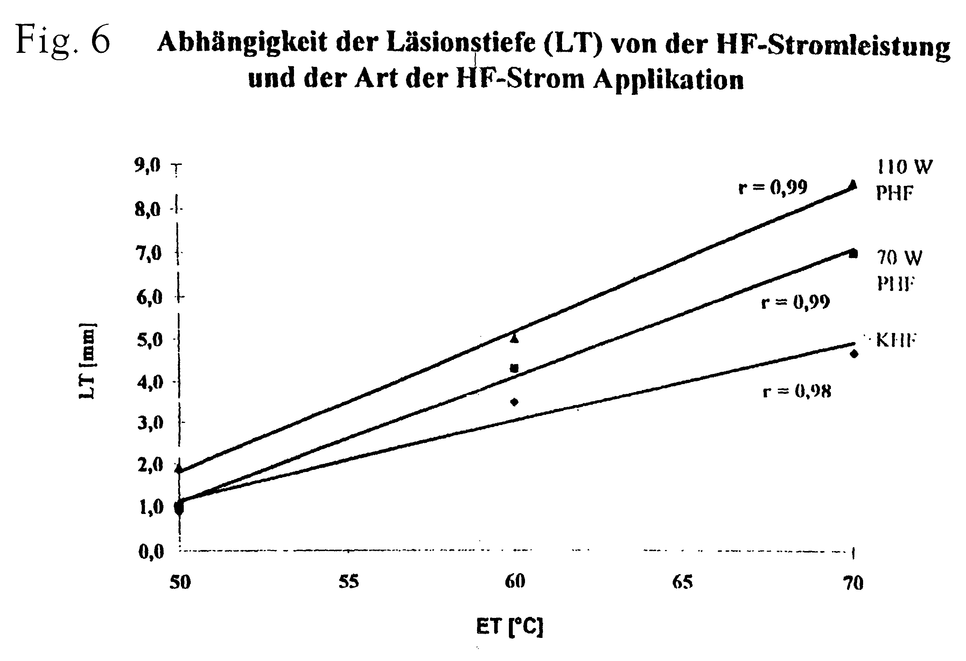

In Fig. 6 sind die Läsionstiefen in Abhängigkeit von der maximal erlaubten Elektrodentemperatur und der Art der Anlegung der HF-Leistung dargestellt. Die Kurve mit der geringsten Steigung beschreibt das Verhalten bei einer Einspeisung einer kontinuierlichen HF-Stromleistung (KHF) von 70 Watt. Im Gegensatz dazu zeigt die Kurve mit der mittleren Steigung den Verlauf bei einer Anwendung einer gepulsten HF-Stromleistung (PHF) von 70 Watt. Die gepulste Arbeitsweise ermöglicht somit im Vergleich zur kontinuierlichen Anwendung eine wesentlich größere Läsionstiefe.

Mit der gepulsten Betriebsart ist die Einspeisung einer HF-Stromleistung von 110 Watt bei einer Kathetertemperatur von etwa 60 °C möglich, welches große Läsionstiefen ermöglicht. Gemäß der Erfindung wird bei der Katheterablation im Herzen von lebenden Patienten eine vorzugsweise zwischen 40°C und 90°C, in besonders bevorzugter Weise zwischen 50°C und 65°C und in bevorzugtester Weise bei 65°C liegende Temperatur im Gewebe erzeugt. Hierbei überschreitet bei der bevorzugtesten Ausführungsform der Erfindung die Kathetertemperatur eine zwischen 40°C und 70°C liegende Grenze nicht.

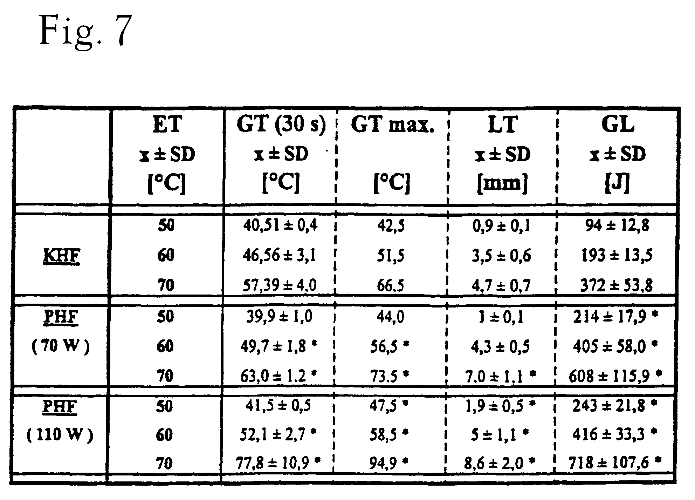

Messungen ergaben, daß bei einer Leistung von 70 Watt etwa doppelt soviel Energie an das zu abladierende Gewebe beim Pulsbetrieb im Vergleich zum kontinuierlichen Betrieb abgegeben wird. Diesbezüglich sei auch auf die Meßwerte der Fig. 7 verwiesen, welche bei dem kontinuierlichen Verfahren (KHF) und dem gepulsten Verfahren (PHF) die Gewebeinnentemperatur jeweils gemittelt über die letzten 30 s der Ablation (GT(30s)), die maximale Gewebeinnentemperatur (GTmax), die erreichte Läsionstiefe (LT) und die hierbei abgegebene Gesamtleistung (GL; entsprechend der integrierten momentanen Leistung) darstellt.

Der mit der beschriebenen Aparatur gemessene Wertesatz wird nachfolgend während der Ablation zum Ermitteln der Gewebeinnentemperatur verwendet. Hierzu wird während des Ablationsvorgangs in einer in den Figuren nicht dargestellten zugeordneten Meß- und Steuereinrichtung die abgegebene Leistung und die Katheterelektrodentemperatur erfasst und daraus die Gewebeinnentemperatur berechnet.

Es werden mittels der Fließgeschwindikeitssensoren 4 auch die momentanen oder zeitlich gemittelten Fließgeschwindigkeiten erfasst und zur genaueren Berechnung der Gewebeinnentemperatur mitberücksichtigt.

Generell kann mit der Erfindung die Kathetertemperatur auf einen, vorzugswewise koagulationsfreien maximalen Wert beschränk werden und die Zeit erfasst werden, nach welcher eine maximale Gewebeinnentemperatur erreicht wurde. Die maximale Gewebeinnentemperatur kann fern von gefährlichen Werten gewählt und einer gewünschten Läsionstiefe zugeordnet werden.

Es liegt im Rahmen der Erfindung, die gemessenen sowie die jeweils momentan berechneten Parameter mit einer externen Computereineit, insbesondere mit einem Personal Computer (PC) vorzunehmen, die/der mit der HF-Steuereinrichtung kommuniziert. Hierbei kann der Bediener am PC die erwünschte örtlich zugeordnete Läsionstiefe eingeben und entweder festlegen, daß die Abgabe der HF-Leistung an die jeweilige Elektrode automatisch beendet oder vermindert wird, oder daß dem behandelden Arzt das Erreichen der Läsionstiefe optisch an einem Monitor oder akkustisch angezeigt wird, wobei in letzterem Falle die weitere Ablation dem behandelnden Arzt obliegt, der diese unter Berücksichtigung von EKG-Daten fortsetzen kann.

Weiterhin hat es sich gezeigt, daß mit der gepulsten Energieabgabe eine wesentlich gleichförmigere Läsion erzeugt werden kann. Ebenso tritt Vaporisation, d.h. die Bildung von Dampfblasen, wesentlich seltener auf, als bei der kontinuierlichen HF-Abgabe. Dies ist auf eine wesentlich gleichmäßigere Temperaturverteilung im Gewebe zurückzuführen. Insbesondere müssen bei der gepulsten Energieabgabe die Gewebeteile bis in ca 1mm bis 2mm Tiefe nicht so stark erwärmt werden, um tiefe Läsionen, insbesondere Läsionen mit einer Tiefe von mehr als 6mm zu erzeugen.

## Patentansprüche

1. Vorrichtung zur Katheterablation umfassend einen Ablationskatheter und einen diesem zugeordneten Anschluß an einen Hochfrequenzgenerator, an ein gesteuertes oder an ein geregeltes Hochfrequenzablationsgerät,
wobei der Katheter zumindest eine Elektrode zum Abladieren von Gewebe durch Abstrahlen von abladierender Leistung, vorzugsweise von Hochfrequenzleistung und vorzugsweise zumindest einen dieser Elektrode zugeordneten thermischen Sensor zur Erfassung der Temperatur der Elektrode aufweist,
eine Einrichtung zum Erfassen eines Parameters der mit der Temperaturdifferenz zwischen der an der Elektrode gemessenen Temperatur und der Temperatur im Gewebe in der Nähe der Elektrode in Beziehung steht, **gekennzeichnet dadurch dass** der Katheter zumindest eine Einrichtung zur Erfassung der momentanen lokalen oder der momentan örtlich gemittelten Fließgeschwindigkeit des Blutes in der Umgebung der Elektrode aufweist.

2. Vorrichtung nach Anspruch 1, **dadurch gekennzeichnet, daß** der erfasste Parameter die in das Gewebe abgegebene Leistung ist und die Einrichtung zum Erfassen eine Leistungserfassungseinrichtung ist, wobei die Ablation im wesentlichen bei vorgegebener und geregelter Kathetertemperatur durchführbar ist.

3. Vorrichtung nach Anspruch 1 oder 2, **dadurch gekennzeichnet, daß** der Katheter mehrere Elektroden und mehrere, den jeweiligen Elektroden zugeordnete thermische Sensoren umfaßt, die im wesentlichen die Temperatur der jeweiligen Elektrode erfassen.

4. Vorrichtung nach Anspruch 1, 2 oder 3, **dadurch gekennzeichnet, daß** die jeweilige Elektrode in Abhängigkeit von der Elektrodentemperatur und der vorher abgegebenen Energie in Form von Pulsen mit fester oder variabler Länge derart angesetuert wird, daß im zu abladierenden Gewebe im wesentlichen eine vorgegebbar einstellbare Temperatur erreicht wird und wobei vorzugsweise die Elektrodentemperatur eine vorgegebene Grenztemperatur nicht überschreitet.

5. Vorrichtung nach einem der vorstehenden Ansprüche, **dadurch gekennzeichnet, daß** die Gewebeinnentemperatur als Funktion der Kathetertemperatur sowie als Funktion der abgegebenen Leistung und eines vorher bestimmten Wertesatzes, der die Temperaturverhältnisse im Gewebe relativ zur Kathetertemperatur für definierte abgegebene Leistungen angbibt, ermittelt wird.

6. Vorrichtung nach Anspruch 7,
**dadurch gekennzeichnet, daß** die Elektrode ferner in Abhängigkeit von der Fließgeschwindigkeit des Blutes in der Umgebung der Elektroden angesteuert werden, so daß im zu abladierenden Gewebe eine vorgegebene Temperatur als Funktion der Kathetertemperatur, der abgegebenen Leistung und/oder der Fließgeschwindikeit erreicht wird.

7. Vorrichtung nach einem der Ansprüche 6, 7 oder 8, **dadurch gekennzeichnet, daß** die Elektrode ferner in Abhängigkeit der gewünschten Läsionstiefe angesteuert werden.

8. Vorrichtung nach einem der vorstehenden Ansprüche, **dadurch gekennzeichnet, daß** die Elektroden vorzugsweise entlang einer ununterbrochenen Linie mit einer Länge von bis zu 7 cm jeweils durch Isolationsbereiche voneinander getrennt, vorzugsweise am Ende des Ablationskatheters, angeordnet sind.

9. Vorrichtung nach einem der vorstehenden Ansprüche, **dadurch gekennzeichnet, daß** die Elektroden in Form einer Pulsbreitenmodulation angesteuert werden.

10. Ablationskatheter mit zumindest einer Ablationselektrode (2), wobei der Katheter zumindest einen der Elektrode 2 zugeordneten thermischen Sensor (3) oder einen Fließgeschwindigkeitssensor (4) umfaßt, wobei der Katheter (1) jeweilige Zuleitungen der Sensoren zu Meß- und Steuereinrichtungen sowie zu einem Hochfrequenzgenerator, einem gesteuerten oder geregelten Hochfrequenzablationsgerät umfaßt, **gekennzeichnet dadurch dass** der Katheter zumindest eine Einrichtung zur Erfassung der momentanen Lokalen oder der örtlich gemittelten Fließgeschwindigkeit des Blutes in der Umgebung der Elektrode aufweist.

## Claims

1. A device for catheter ablation comprising an ablation catheter and, associated therewith, a connection to a high-frequency generator, to an open or closed loop controlled high-frequency ablation apparatus, the catheter comprising at least one electrode for the ablation of tissue by emitting radiation of a power adapted to ablation, preferably high-frequency power, and preferably at least one thermal sensor associated with said electrode for detecting the temperature of the electrode, a device for detecting a parameter which is related to the temperature difference between the temperature measured at the electrode and the temperature in the tissue near the electrode, **characterised in that** the catheter comprises at least one device for detecting the instantaneous local or instantaneously locally averaged speed of flow of the blood in the surroundings of the electrode.

2. A device according to claim 1, **characterised in that** the detected parameter is the power delivered into the tissue and the detection device is a power detection device, the ablation being performable substantially at a predetermined and regulated catheter temperature.

3. A device according to claim 1 or 2, **characterised in that** the catheter comprises a plurality of electrodes and a plurality of thermal sensors which are associated with the respective electrodes and which detect substantially the temperature of the respective electrode.

4. A device according claim 1, 2 or 3, **characterised in that** the respective electrode is so activated in dependence on the electrode temperature and the previously delivered energy in the form of pulses of fixed or variable length that a presettable temperature is substantially obtained in the tissue for ablation, the electrode temperature preferably not exceeding a predetermined limit temperature.

5. A device according to any one of the preceding claims, **characterised in that** the tissue internal temperature is determined as a function of the catheter temperature and as a function of the delivered power and of a predetermined set of values indicating the temperature conditions in the tissue relative to the catheter temperature for specific delivered powers.

6. A device according to claim 7, **characterised in that** the electrode is also activated in dependence on the speed of flow of the blood in the surroundings of the electrodes so that a predetermined temperature as a function of the catheter temperature, the delivered power and/or the speed of flow is obtained in the tissue for ablation.

7. A device according to any one of claims 6, 7 or 8, **characterised in that** the electrodes are also activated in dependence on the required lesion depth.

8. A device according to any one of the preceding claims, **characterised in that** the electrodes are disposed preferably along an uninterrupted line of a length of up to 7 cm each separated from one another by insulation zones, preferably at the end of the ablation catheter.

9. A device according to any one of the preceding claims, **characterised in that** the electrodes are activated in the form of a pulse width modulation.

10. An ablation catheter with at least one ablation electrode (2), the catheter comprising in association with the electrode (2) at least one thermal sensor (3) or a speed of flow sensor (4), the catheter (1) comprising respective lines leading from the sensors to measuring and control devices and to a high-frequency generator, an open or closed loop controlled high-frequency ablation apparatus, **characterised in that** the catheter comprises at least one device for detecting the instantaneous local or the locally averaged speed of flow of the blood in the surrounding of the electrode.

## Revendications

1. Dispositif destiné à l'ablation par cathéter, comportant un cathéter d'ablation et, associé à celui-ci, un raccordement à un générateur haute fréquence, à un appareil d'ablation haute fréquence commandé ou régulé,
le cathéter comportant au moins une électrode pour l'ablation de tissu par rayonnement de puissance d'ablation, de préférence de puissance haute fréquence, et de préférence au moins un détecteur thermique associé à cette électrode pour la détection de la température de l'électrode,
un dispositif pour la détection d'un paramètre qui est en relation avec la différence de température entre la température mesurée au niveau de l'électrode et la température dans le tissu à proximité de l'électrode,
**caractérisé en ce que** le cathéter comporte au moins un dispositif pour la détection de la vitesse momentanée locale ou de la vitesse momentanée locale moyenne de la circulation du sang dans l'environnement de l'électrode.

2. Dispositif selon la revendication 1, **caractérisé en ce que** le paramètre détecté est la puissance délivrée au tissu, et **en ce que** le dispositif de détection est un dispositif de détection de puissance, l'ablation pouvant pour l'essentiel être effectuée à une température de cathéter prédéterminée et régulée.

3. Dispositif selon la revendication 1 ou 2, **caractérisé en ce que** le cathéter comporte plusieurs électrodes, et plusieurs détecteurs thermiques associés aux électrodes respectives qui détectent pour l'essentiel la température des électrodes respectives.

4. Dispositif selon la revendication 1, 2 ou 3, **caractérisé en ce que**, en fonction de la température d'électrode et de l'énergie délivrée auparavant, chaque électrode est commandée sous forme d'impulsions de durée fixe ou variable de telle sorte qu'une température pouvant être prédéterminée et régulée soit pour l'essentiel atteinte dans le tissu à ablater, et la température d'électrode ne dépassant pas de préférence une température limite prédéterminée.

5. Dispositif selon l'une des revendications précédentes, **caractérisé en ce que** la température interne du tissu est déterminée en tant que fonction de la température du cathéter, ainsi qu'en tant que fonction de la puissance délivrée et d'un taux de valeur déterminé auparavant, qui indique les relations de température dans le tissu par rapport à la température du cathéter pour des puissances définies délivrées.

6. Dispositif selon la revendication 7, **caractérisé en ce que** les électrodes sont en outre commandées en fonction de la vitesse de circulation du sang dans l'environnement des électrodes, de sorte qu'une température prédéterminée soit atteinte dans le tissu à ablater en tant que fonction de la température du cathéter, de la puissance délivrée et/ou de la vitesse de circulation.

7. Dispositif selon l'une des revendications 6, 7 ou 8, **caractérisé en ce que** les électrodes sont en outre commandées en fonction de la profondeur de lésion souhaitée.

8. Dispositif selon l'une des revendications précédentes, **caractérisé en ce que** les électrodes sont de préférence disposées le long d'une ligne ininterrompue d'une longueur pouvant aller jusqu'à 7 cm, en étant respectivement séparées les unes des autres par des zones d'isolation, de préférence au niveau de l'extrémité du cathéter d'ablation.

9. Dispositif selon l'une des revendications précédentes, **caractérisé en ce que** les électrodes sont commandées sous la forme d'une modulation d'impulsions en durée.

10. Cathéter d'ablation avec au moins une électrode d'ablation (2), le cathéter comportant au moins un détecteur thermique (3) associé à l'électrode (2) ou un détecteur de vitesse de circulation (4), et le cathéter (1) comportant des lignes d'amenée respectives des détecteurs vers des dispositifs de mesure et de commande, ainsi que vers un générateur haute fréquence, un appareil d'ablation haute fréquence commandé ou régulé, **caractérisé en ce que** le cathéter comporte au moins un dispositif pour la détection de la vitesse locale instantanée ou de la vitesse locale moyenne de circulation du sang dans l'environnement de l'électrode.
